# EUROPEAN PATENT SPECIFICATION

(11) **EP 2 470 232 B1**
(45) Date of publication and mention of the grant of the patent: **30.03.2016**
(21) Application number: 10745712.9
(22) Date of filing: 18.08.2010
(51) Int. Cl.: A61L 29/14, A61L 29/00, A61L 27/50, A61M 25/10, A61M 25/00, A61L 29/16

(54) **BALLOON CATHETER DEVICES WITH DRUG-COATED SHEATH**
BALLONKATHETERVORRICHTUNGEN MIT WIRKSTOFFBESCHICHTETER SCHLEUSE
DISPOSITIFS DE CATHÉTER À BALLONNET PRÉSENTANT UNE GAINE ENROBÉE DE MÉDICAMENT

(30) Priority: 27.08.2009 US 237437 P; 30.12.2009 US 291100 P
(43) Date of publication of application: 04.07.2012
(73) Proprietor: Boston Scientific Scimed, Inc., Maple Grove, MN 55311 (US)
(72) Inventor: KANGAS, Steve, Woodbury, Minnesota 55125 (US); RIZQ, Raed, Maple Grove, Minnesota 55311 (US); SUTERMEISTER, Derek, Eden Prairie, Minnesota 55346 (US)
(74) Representative: Peterreins Schley
(86) International application number: PCT/US2010/045877
(87) International publication number: WO 2011/028419

(56) References cited:
- EP-A1- 0 712 615
- EP-A2- 1 595 569
- WO-A1-2009/046206
- WO-A1-2011/009096
- WO-A2-03/011363
- WO-A2-2005/027996
- US-A- 5 102 402
- US-A- 5 634 901
- US-A- 5 645 789
- US-A- 6 027 486
- US-A1- 2008 033 477
- US-A1- 2008 140 002

## Description

### TECHNICAL FIELD

The present invention relates to medical devices, more particularly, to balloon catheter devices.

### BACKGROUND

Balloon catheters are used in a wide variety of minimally-invasive or percutaneous medical procedures. Balloon catheters having drug coatings may be used to treat diseased portions of blood vessels. Typically, the drug-coated balloon is inserted through a peripheral blood vessel and then guided via a catheter through the vascular system to the target intravascular site. At the target site, the balloon is inflated and the drug is applied to the blood vessel. However, there may be problems with releasing the drug from the balloon. For example, there may be insufficient fragmentation of the drug coating or the drug coating may not sufficiently delaminate off of the balloon.

US 2008/1400002 A1 describes a drug delivery system using a balloon having various architectures to increase the capacity of such a system for carrying a drug.

Therefore, there is a need for improved balloon catheter devices for drug delivery to an intravascular site.

### SUMMARY

The present invention is directed to a balloon catheter device with a drug-coated sheath defined in claim 1. The dependent claims depict preferred embodiments of the present invention.

In one embodiment, the present invention provides a medical device comprising: (a) a balloon; (b) an expandable sheath disposed around the balloon; and (c) a coating disposed over the expandable sheath, the coating comprising a therapeutic agent, and wherein the coating is less compliant than the expandable sheath. In one aspect, the invention is directed to a retaining ring that has not been use in device substrate polishing. The retaining ring has a generally annular body having a top surface, an inner diameter surface, an outer diameter surface and a bottom surface. The bottom surface has a target surface characteristic that substantially matches an equilibrium surface characteristic that would result from breaking-in the retaining ring with the device substrate polishing.

In another embodiment, the present invention provides a medical device comprising: (a) a balloon having a balloon wall, the balloon wall comprising an outermost layer and an inner layer, the outermost layer being less compliant than the inner layer; and (b) a coating disposed over the outermost layer, the coating comprising a therapeutic agent.

In another embodiment, the present invention provides a method of making a medical device, comprising: (a) providing a balloon having a polymer wall; (b) embrittling the outer surface of the polymer wall to form an outermost layer that is less compliant than the rest of the polymer wall; and (c) disposing a coating over the outermost layer, wherein the coating comprises a therapeutic agent.

### BRIEF DESCRIPTION OF DRAWINGS

FIGS. 1A and IB show a sheath-covered balloon catheter device according to one embodiment of the present invention. The device is shown with the balloon in an uninflated state. FIG. 1A shows a side view of the catheter device, and FIG. IB shows a magnified transverse cross-section view of the catheter device.
FIGS. 2A and 2B show the balloon catheter device of FIG. 1 in an inflated state. FIG. 2A shows a side view of the catheter device, and FIG. 2B shows a magnified transverse cross-section view of the catheter device.
FIG. 3 shows a microscopic image of paclitaxel particles on an elastic sheet.
FIGS. 4A and 4B show the results of an experimental trial using a clear polyurethane tube as a model for a blood vessel.
FIGS. 5A - C show an example of a balloon catheter device, which does not form part of the present invention. FIG. 5A shows the device with the balloon uninflated. FIG. 5B shows the device with the balloon at an intermediate stage of inflation. FIG. 5C shows the device with the balloon fully inflated.
FIGS. 6A - C are a sequence of photos showing examples of a balloon at various stages of inflation. FIG. 6A shows an example of the balloon uninflated. FIG. 6B shows an example of the balloon at an intermediate stage of inflation. FIG. 6C shows an example of the balloon fully inflated.
FIGS. 7 A and 7B show transverse cross-section views of an example of a balloon catheter device, which does not form part of the present invention. FIG. 7A shows an example of the balloon before UV-ray treatment, and FIG. 7B shows an example of the balloon after UV ray treatment.
FIGS. 8A and 8B show examples of magnified views of a portion of the wall of the balloon shown in FIG. 7B. FIG. 8A shows an example of the balloon wall before the balloon is inflated, and FIG. 8B shows an example of the balloon wall after the balloon is inflated.

### DETAILED DESCRIPTION

Medical devices of the present invention have an inflatable balloon for delivering a therapeutic agent to a target site in a patient's body. The balloon is designed to be insertable in the body using any of various mechanisms conventionally used for the delivery, actuation, or inflation of balloon devices. The balloon device may be designed similar to those that have been known in the art, including but not limited to angioplasty catheters, stent delivery catheters, inflation catheters, and/or perfusion catheters. The medical devices of the present invention may be used in conjunction with other intravascular drug delivery devices, such as vascular stents.

In one embodiment of the present invention, an expandable sheath is disposed around the balloon. The expandable sheath may be made of various types of elastomeric or expandable materials, such as silicone elastomers, fluoropolymer elastomers, or thermoplastic elastomers. Examples of thermoplastic elastomers include thermoplastic polyurethanes, thermoplastic polyesters, and thermoplastic polyamides such as polyether block amide (e.g., PEBAX®). Examples of fluoropolymer elastomers include polymers or copolymers of tetrafluoroethylene, hexafluoropropylene, or vinylidene fluoride. The expandable sheath mayor may not have any attachments to the surface of the balloon (e.g., the sheath may without such attachments and be "free-floating" over the balloon surface). The expandable sheath mayor may not be elastic (i.e., the deformation of the sheath upon expansion mayor may not be reversible).

The balloon may have varying degrees of compliance, depending upon the particular application. For example, the balloon may be a compliant, non-compliant, or a semi-compliant balloon. As used herein, a "non-compliant balloon" means a balloon whose diameter increases by no more than 10% of the rated nominal diameter as the internal pressure in the balloon is increased above the nominal inflation pressure. As used herein, a "semi-compliant balloon" means a balloon whose diameter increases by no more than 20% of the rated nominal diameter as the internal pressure in the balloon is increased above the nominal inflation pressure. As used herein, a "compliant balloon" means a balloon whose diameter increases by more than 20% of the rated nominal diameter as the internal pressure in the balloon is increased above the nominal inflation pressure. For coronary artery balloons, nominal diameters may range from 1.5 - 7.0 mm, and in the most typical cases, from 2.0 - 4.0 mm. However, other nominal balloon diameters are also possible, depending upon the intended target site and/or the particular application.

A coating containing a therapeutic agent is disposed over the expandable sheath. The coating may be the therapeutic agent alone, or the therapeutic agent in combination with one or more other materials. For example, the therapeutic agent can be blended with additives or excipient materials (e.g., binders, plasticizers, fillers, etc.) to make the coating more or less brittle. In any case, the coating of therapeutic agent is formulated to be less compliant than the expandable sheath. As such, the coating of therapeutic agent will break apart as the sheath expands. The thickness of the coating will vary depending upon the application, and in some cases, the coating thickness is in the range of 1 - 10 !lm. Thinner or thicker coatings are also possible.

In certain embodiments, the therapeutic agent may be provided in a crystalline form. For some therapeutic agents, such as paclitaxel, the crystalline form is less soluble and has a coarser, grainier texture than the amorphous form. This may allow the therapeutic agent to adhere better to the blood vessel wall, improve tissue penetration, and/or become less susceptible to washing downstream after transfer to the blood vessel wall.

Upon inflation of the balloon, the unfolding and/or expansion of the balloon will exert outward pressure on the sheath, causing the expandable sheath to expand as well. The sheath may expand in a radial direction, longitudinal direction, a combination thereof, or any other direction. Where radial expansion is involved, the amount of radial expansion that the expandable sheath experiences as the balloon is inflated will vary with different balloon devices. In some cases, the balloon device is designed such that the sheath experiences at least a 1.5-fold increase in diameter (i.e., at least 50% radial elongation), and in some cases, at least a 2.5-fold increase in diameter (i.e., at least 150% radial elongation) as the balloon is inflated from its uninflated state. However, other amounts of radial expansion are also possible, depending upon the particular application. Because the therapeutic agent coating on the sheath is less compliant than the sheath, the coating will break into fragments (e.g., particles) as the sheath expands. The fragments of therapeutic agent may be applied onto the body tissue and/or released from the balloon.

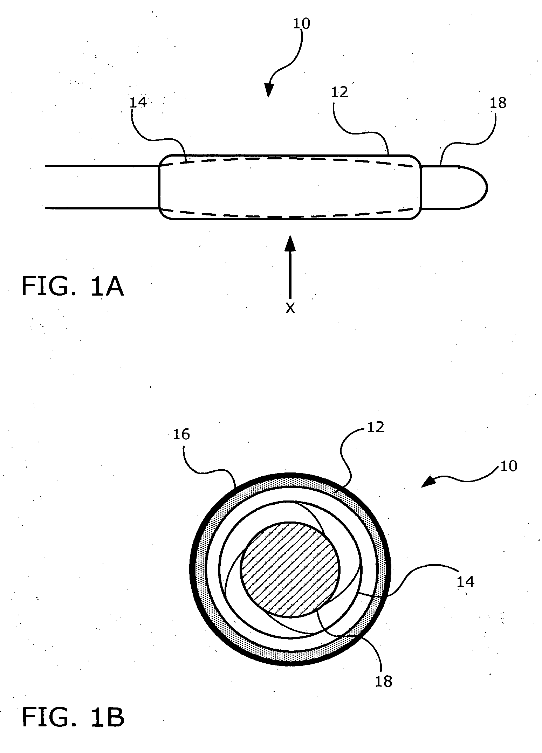

FIGS. 1A and 1B show a balloon catheter device 10 according to one embodiment of the present invention. FIG. 1A shows a side view of catheter device 10, and FIG. IB shows a magnified transverse cross-section view of catheter device 10 along plane X in FIG. 1A. Balloon catheter device 10 comprises a non-compliant balloon 14 mounted on a flexible catheter shaft 18. Balloon 14 is covered by an expandable sheath 12. As seen in FIG. IB, balloon 14 is folded into a compact configuration within sheath 12. Sheath 12 has a coating 16 (not shown in FIG. 1A) that contains a therapeutic agent. Sheath 12 may be fixed onto balloon 14 at the proximal and distal points of sheath 12 (e.g., by spot welding or tacking) to help re-fold and/or retain sheath 12 on balloon 14 when balloon 14 is deflated for withdrawal. Sheath 12 may be elastic and may have a resting diameter smaller than folded balloon 14 so that the sheath 12 contracts around balloon 14.

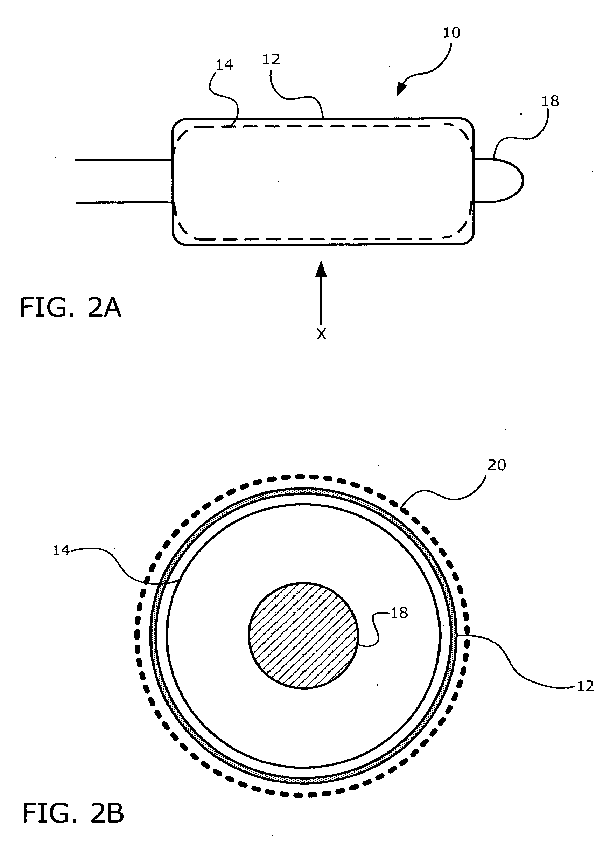

In operation, with balloon 14 in an uninflated condition (as shown in FIGS. 1A and 1B), balloon 14 is inserted into a patient's body using catheter shaft 18. At the target site, as shown in FIGS. 2A and 2B, balloon 14 is inflated. FIG. 2A shows a side view of catheter device 10, and FIG. 2B shows a magnified transverse cross-section view of the catheter device 10 along plane X in FIG. 2A. As balloon 14 is inflated, it unfolds and expands radially, causing sheath 12 to expand radially with it. (For clarity in illustration, sheath 12 is shown not touching balloon 14, but it will be understood that sheath 12 may contact balloon 14 or that an intermediate material may be interposed between sheath 12 and balloon 14.)

As shown in FIG. 2B, with the radial expansion of sheath 12, therapeutic agent coating 16 is made to break apart into particles 20 (not shown in FIG. 2A). Particles 20 may then delaminate off of sheath 12 and become released and/or applied directly onto the body tissue, e.g., a blood vessel wall. The size of particles 20 could be modified by blending the therapeutic agent with excipient materials, such as binders, plasticizers, or fillers. For example, by mixing the therapeutic agent with binders, therapeutic agent coating 16 could be made to break apart into larger size particles. Alternatively, mixing the therapeutic agent with other types of excipient materials could cause coating 16 to break apart into smaller particles. In some cases, to facilitate delamination of therapeutic agent coating 16 and release of particles 20, sheath 12 could be made using a low adhesion material, such as low-adhesion silanes or the above described fluoropolymer elastomers. The size of particles 20 could also be modified by scoring or patterning of the therapeutic agent coating 16.

In an alternate embodiment, there is an intermediate release layer between sheath 12 and therapeutic agent coating 16 that facilitates the delamination of therapeutic agent coating 16 off of sheath 12 as coating 16 breaks apart into particles 20. The intermediate release layer can be made in various ways to perform this function. For example, the release layer may comprise a low adhesion material, such as low-adhesion silanes or the above-mentioned fluoropolymer elastomers. In another example, the release layer may comprise a material that dissolves or degrades upon exposure to body fluids (e.g., a sugar or biodegradable polymer). In another example, the release layer may comprise a material that absorbs fluid and swells upon exposure to body fluids (e.g., a hydrogel). In each case, the release layer facilitates the delamination and release of the fragments of therapeutic agent.

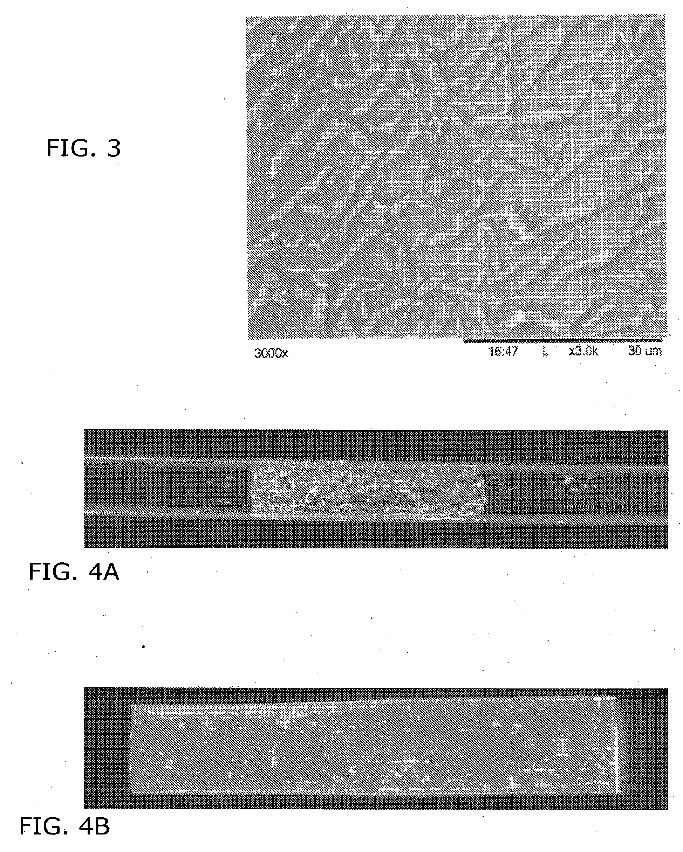

FIG. 3 shows a microscopic image of paclitaxel particles on an elastic sheet made of polyvinylidene fluoride-hexafluoropropylene 85:15 wt% copolymer. To produce this image, the elastic sheet was coated with a solution of paclitaxel in tetrahydrofuran. The coating solution was then dried to form a continuous glassy film of paclitaxel on the elastic sheet. The sheet was then subjected to more than 200% elongation by stretching in one direction. As shown in FIG. 3, this resulted in the paclitaxel film breaking apart into small particles and delaminating off of the elastic sheath. This result demonstrates that a therapeutic agent coating disposed over an expandable sheath in the manner of the present invention can allow for fragmentation of the coating into particles that can more easily detach from the sheath and/or be more easily absorbed by body tissue.

FIGS. 4A and 4B show the results of an experimental trial using a clear polyurethane tube as a model for a blood vessel. A coating solution was made by mixing paclitaxel and polyvinylpyrolidinone (in a 80/20 wt/wt ratio) in THF at a 15 wt% concentration. A tubular sheath (outer diameter = 0.071 inches, wall thickness = 0.0050 inches) made of elastomeric silicone was coated by dipping into the paclitaxel PVP solution and drying for 20 minutes under vacuum at room temperature. The resulting coating provided a paclitaxel loading of about 3µg/mm². To convert the amorphous paclitaxel into crystalline form by vapor annealing, the coated sheath was then placed in a chamber saturated with ethanol vapor (180 proof) for 24 hours.

The coated elastic sheath was mounted on a 3.0 mm x 20 mm angioplasty balloon from a Liberte™ stent system (Boston Scientific). The balloon/sheath was inserted into the hydrophilic polyurethane tube in a water bath at 37° C. The balloon was held in the polyurethane tube for 1 minute and then inflated. The polyurethane tube was sized to give 20% overstretch during balloon deployment. The balloon was maintained in the inflated state for 1 minute, vacuum was pulled for 15 seconds, and the balloon/sheath was withdrawn from the polyurethane tube. The polyurethane tube was then removed from the water bath, dried, and imaged.

FIG. 4A shows an image (5x magnification) of the polyurethane tube after balloon deployment of the sheath and withdrawal from the polyurethane tube. This image demonstrates that a significant amount of the drug coating was transferred from the sheath to the inner surface of the polyurethane tube. FIG. 4B shows an image (10x magnification) of the elastic sheath after deployment and withdrawal from the polyurethane tube. This image demonstrates that most of the drug coating was transferred and only a small amount of the drug coating (seen as white particles) remains on the elastic sheath.

During the balloon deployment process, fluid flow around the balloon may wash the therapeutic agent coating on the expandable sheath downstream. Due to the increased fluid velocity around the balloon as the flow volume shrinks with balloon expansion, a considerable portion of this loss may occur during balloon expansion at moments just prior to when the balloon contacts the wall of the blood vessel.

As such, the medical device may be designed to reduce this loss of therapeutic agent coating during balloon expansion. In certain examples, the length of the expandable sheath is shorter than the length of the balloon such that one or both ends of the balloon is unconstrained by the sheath. As used herein, the length of the balloon and sheath refers to the length as measured when the balloon is in the nominally inflated state. During expansion of the balloon, this configuration can allow for the unconstrained end(s) of the balloon to begin inflating before the sheath-covered portion of the balloon. The sheath can have various lengths relative to the balloon length. For example; in certainexamples, the sheath can be 20 - 80% of the length of the balloon, but other lengths are also possible. One or both ends of the balloon may be left uncovered by the sheath.

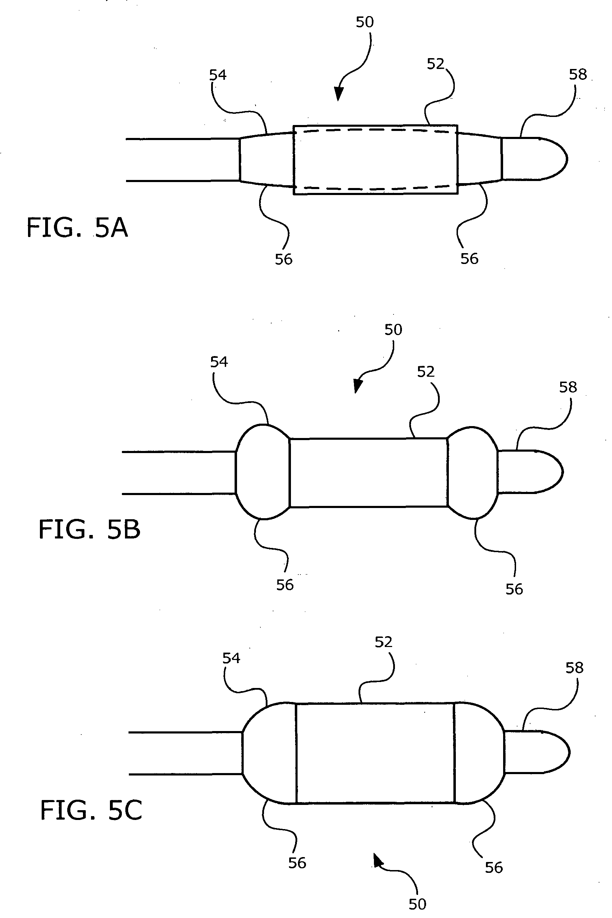

For example, FIGS. 5A - C show examples of a balloon catheter device 50, which do not form part of the present invention. As seen in FIG. 5A, balloon catheter device 50 comprises a balloon 54 mounted on a flexible catheter shaft 58. The midsection of balloon 54 is covered by an expandable sheath 52. (For clarity in illustration, sheath 52 is shown not touching balloon 54, but it will be understood that sheath 52 may contact balloon 54 or that an intermediate material may be interposed between sheath 52 and balloon 54.) The end portions 56 of balloon 54 are not covered by sheath 52. Sheath 52 has a coating that contains a therapeutic agent (not shown). Sheath 52 may be elastic and may have a resting diameter smaller than balloon 54 in a folded configuration so that the sheath 52 contracts around balloon 54.

In operation, with balloon 54 in an uninflated state, balloon 54 is inserted into a blood vessel using catheter shaft 58. At the target site, balloon 54 is inflated. As seen in FIG. 5B, at the early stages of inflation (e.g., at 1 atm pressure), inflation begins at the unconstrained end portions 56 of balloon 54, which form inflated lobes that cause balloon 54 to take on a "dumbbell" shape. As the end portions 56 of balloon 54 contact the blood vessel wall, they can restrict the flow of blood around balloon 54 to protect the therapeutic agent coating on sheath 52 from being washed away. Furthermore, if portions of the therapeutic agent coating detach from sheath 52 during balloon inflation, the therapeutic agent may be trapped between the lobes at end portions 56 instead of being washed downstream with the flow of blood. As balloon 54 is further inflated, its midsection begins to expand radially, causing sheath 52 to expand radially with it. As shown in FIG. 5C, when balloon 54 fully inflated (e.g., at an inflation pressure of 11 atm), sheath 52 applies the therapeutic agent coating against the blood vessel wall.

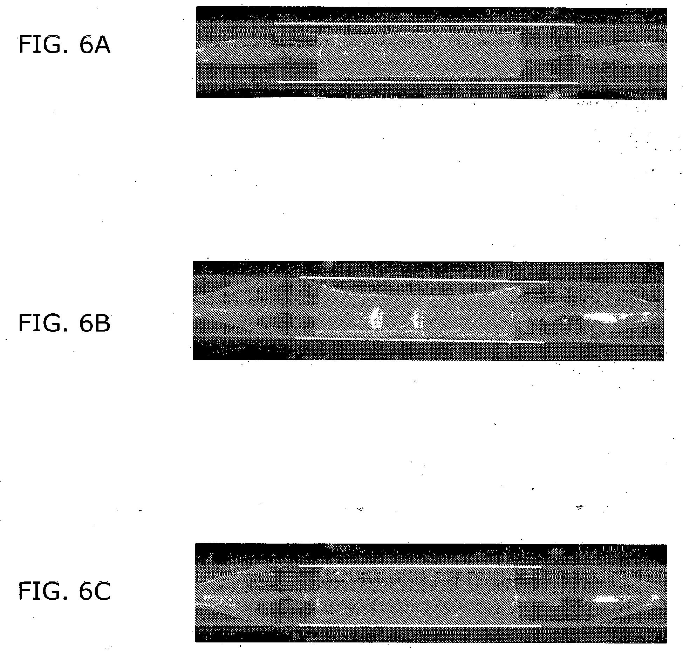

FIGS. 6A - C are a sequence of photos showing examples of a balloon at various stages of inflation inside a clear polyurethane tube (the tube edges are highlighted with a white line to enhance visibility), which do not form part of the present invention. FIG. 6A shows the balloon prior to inflation. As seen here, a sheath that is shorter than the balloon is mounted on the midsection of the balloon such that the ends of the balloon are not covered by the sheath. FIG. 6B shows the balloon at an intermediate stage of inflation where the unconstrained ends begin to inflate before the midsection of the balloon, which is constrained by the sheath. This results in the balloon taking on a "dumbbell" shape because of the inflated lobes formed at its ends. FIG. 6C shows the balloon at full inflation, with the midsection of the balloon now expanded and touching the wall of the polyurethane tube.

In another example, which does not form part of the present invention, the wall of the balloon comprises an outermost layer and one or more inner layers. The outermost layer is less compliant than the inner layer(s) such that cracks will form in the outermost layer as the balloon is inflated to its nominal diameter or beyond. A therapeutic agent is disposed as a coating over the relatively less compliant outermost layer. The coating may be the therapeutic agent alone, or the therapeutic agent in combination with one or more other materials (such as the above-described additives or excipient materials). The coating of therapeutic agent breaks apart when the relatively less compliant outermost layer cracks with the inflation of the balloon.

The outermost layer is sufficiently brittle that the amount of surface area elongation required to cause cracks to form is less than 40% at body temperature (i.e., 37°C) while submerged in a buffered aqueous solution. In other words, up to a 40% increase in the surface area of the outermost layer is sufficient to cause cracks to form in the outermost layer, but this does not mean that the outermost layer necessarily expands to this degree. The amount of brittleness may vary depending upon the compliance characteristics of the balloon. For example, since a non-compliant balloon does not expand as much as a compliant balloon, the outermost layer of the non-compliant balloon may be made to have a higher degree of brittleness (i.e., cracks with less surface area elongation) than for the compliant balloon.

In some cases, the outermost layer has excavated regions to facilitate cracking of the outermost layer. As used herein, "excavated regions" refers to voids (e.g., fracture lines, holes, slots, grooves, channels, etchings, perforations, pits, etc.) that are created by removal of material using techniques that control the size, shape, and location of the voids. For example, such techniques include direct-write etching using energetic beams (e.g., laser, ion, or electron), micromachining, microdrilling, or lithographic processes.

A balloon having a balloon wall with a relatively less compliant outermost layer can be made using any of a number of different techniques known for making multi-layered balloons. One such way is by embrittling the polymer material on the surface of the balloon wall using any suitable embrittling process. Examples of embrittling processes include processes that cross-link the polymer material, processes that cause degradation of the polymer material, or processes that remove any plasticizers. There are various processes for degrading a polymer, such as exposing the polymer to heat, radiation, or reactive chemicals, and the type of process suitable for use will vary depending upon the type of polymer. For example, polyethylene and polypropylene can degrade and become brittle upon oxidation or exposure to ultraviolet (UV) rays. The polymer material may also be degraded by exposure to reactive chemicals, which may be a chemical solution such as a strong acid solution (e.g., sulfuric acid) or a strong base solution. The reactive chemical may also be a reactive gas such as ozone, chlorine, or plasma. For example, polyethylene terephthalates can degrade and become brittle from hydrolysis by strong acids, while polycarbonates can degrade and become brittle when exposed to strong alkalis. Some of these degradative processes involve chain scissioning of the polymers, particularly where the polymer wall is made from long chain polymers.

As mentioned above, embrittling can also be achieved by cross-linking of the polymers in the polymer wall. There are various processes for cross-linking a polymer, and the type of process suitable for use will vary depending upon the type of polymer. Some of the processes for cross-linking include exposing the balloon to heat, pressure, or radiation (such as UV rays, electron beam, or gamma radiation). There may also be photo-initiated cross-linking additives (e.g., benzophenone) in the balloon wall that can facilitate radiation-induced cross linking.

As mentioned above, embrittling can also be achieved by removing plasticizers (e.g., by evaporating or leaching) that may be present in the polymer wall. For example, polyvinyl chloride (PVC) can become brittle with the loss of plasticizers. The coating of therapeutic agent could be applied before or after the above-described embrittling processes. Where the coating of therapeutic agent is applied before the embrittling process, the embrittling process may also serve to trap the therapeutic agent within the outermost layer.

Another way of making a multi-layered balloon having a relatively less compliant outermost layer is by using a co-extrusion process with the outermost layer being made of a different material than the inner layer(s) of the balloon wall. For example, the balloon may be made using the co-extrusion processes described in U.S. Patent No. 5,195,969 (Wang et al.) or U.S. Patent No. 7,166,099 (Devens). The material used in making the outermost layer can differ in a variety of ways from the material used in making the inner layer(s). For example, the outermost layer may be made of a material that is relatively less elastic than the material used in the inner layer(s). In another example, the outermost layer may be made of a material that can be further processed to make the outermost layer less compliant. For example, the outermost layer may be made of a material having additives (e.g., benzophenone) that allow for UV-initiated cross-linking of the polymers in outermost layer.

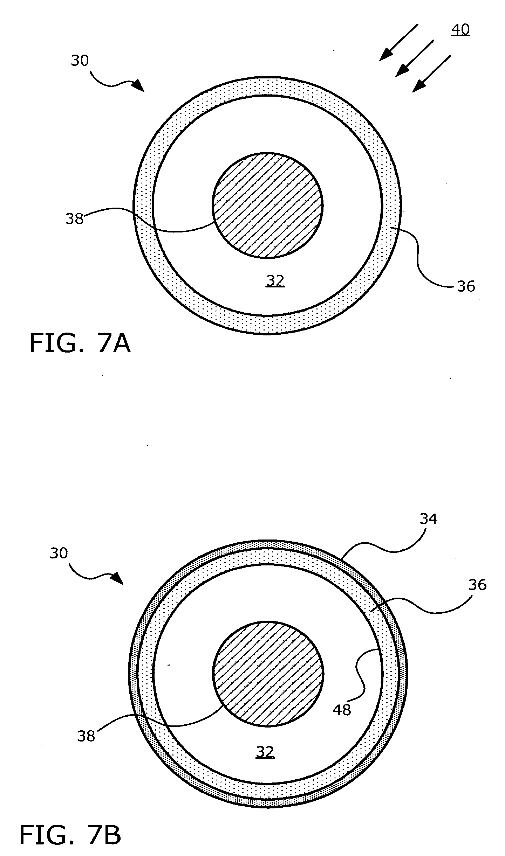

FIGS. 7 A and 7B show transverse cross-section views of an example of a balloon catheter device , which does not form part of the present invention. Referring to FIG. 7 A, the catheter device comprises a semi-compliant balloon 30 mounted on a catheter shaft 38. Balloon 30 has a balloon wall 36 and for illustration purposes only, an exaggerated gap 32 is shown between catheter shaft 38 and balloon wall 36. With balloon 30 nominally inflated, the wall 36 of balloon 30 is irradiated with UVrays 40. To expose the full circumference of balloon wall 36 to the UV -rays 40, balloon 30 is rotated around catheter shaft 38 while being irradiated.

FIG. 7B shows the balloon 30 after UV-ray treatment. The UV-ray treatment has caused the polymer material at the surface of balloon wall 36 to become brittle, creating an outermost layer 34 that is brittle and relatively less compliant than inner layer 48 of balloon wall 36. As explained above, this embrittlement may occur, for example, through UV-induced crosslinking or degradation of the polymers.

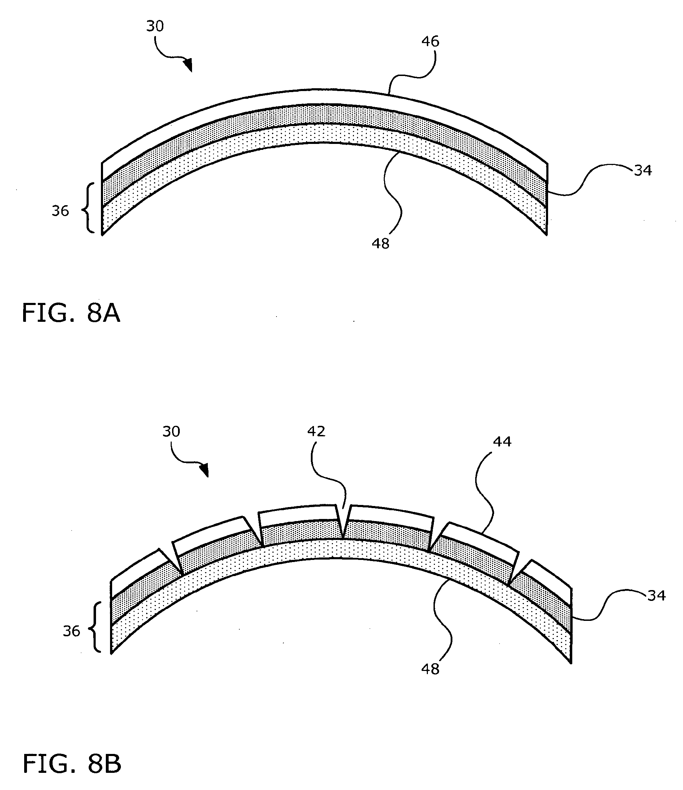

In operation, the catheter device is inserted into a patient's body with the balloon 30 in an uninflated state. FIG. 8A shows a magnified view of a portion of the balloon wall 30 shown in FIG. 7B. FIG. 8A shows the balloon wall 36 having an inner layer 48 and a brittle outermost layer 34, which in tum is coated with a coating 46 containing a therapeutic agent. Referring to FIG. 8B, at the target site, balloon 30 is inflated. With the expansion of balloon wall 36, cracks 42 form in brittle outermost layer 34, which causes cracking and delaminating of therapeutic agent coating 46 into particles 44 that are released into the treatment area. In an alternate examples, which do not form part of the present invention, bilayered balloon 30 shown in FIG. 7B can be made by a co-extrusion process in which outermost layer 34 is made from a different material than inner layer 48. For example, outermost layer 34 can be made of a material that is relatively less elastic than the material used in making inner layer 48.

Examples of additives or excipient materials that can be blended with the therapeutic agent include carbohydrates; polysulfones; physiologically acceptable oils, fats, lipids, lipoids, or waxes; bioresorbable or biodegradable polymers; surfactants such as polyethylene glycol (PEG)fatty acid esters, glycerol fatty esters, or PEG-glyceryl fatty esters; resins such as shellac or its components (e.g., shellolic acid or aleuritic acid); citrate esters such as alkyl acetyl citrates, triethyl acetyl citrate, tributyl acetyl citrate, trihexyl acetyl citrate, alkyl citrates, triethyl citrate, or tributyl citrate; or radiological contrast agents.

Non-limiting examples of carbohydrates include monosaccharides, disaccharides, trisaccharides, oligosaccharides, polysaccharides, and derivatives of sugars (such as sugar alcohols, sugar acids, esterified sugars, and sugar polymers (e.g., Ficoll™)). Examples of sugars include mannitol, sucrose, fructose, mannose, trehalose, and raffinose. Examples of oligosaccharides and polysaccharides include those containing N-acyl glucosamine and uronic acid (e.g., glucuronic acid or iduronic acid) or N-acyl galactosamine and uronic acid.

Non-limiting examples of biodegradable or bioresorbable polymers include polycarboxylic acid, polyanhydrides including maleic anhydride polymers; polyorthoesters; poly-amino acids; polyethylene oxide; polyphosphazenes; polylactic acid, polyglycolic acid and copolymers and mixtures thereof such as poly(L-lactic acid) (PLLA), poly(D,L-lactide), poly(lactic acid-eo-glycolic acid), 50/50 (DL-lactide-co-glycolide); polydioxanone; polypropylene fumarate; polydepsipeptides; polycaprolactone and co-polymers and mixtures thereof such as poly(D,L-lactide-co-caprolactone) and polycaprolactone co-butyl acrylate; polyhydroxybutyrate valerate and blends; polycarbonates such as tyrosine-derived polycarbonates and acrylates, polyiminocarbonates, and polydimethyltrimethylcarbonates; cyanoacrylate; polyglycosaminoglycans; macromolecules such as polysaccharides (including hyaluronic acid; cellulose and hydroxypropyl methyl cellulose; gelatin; starches; dextrans; alginates and derivatives thereof), proteins and polypeptides; and mixtures and copolymers of any of the above.

Contrast agents that can be blended with the therapeutic agent may be suitable for xray imaging, CT scan imaging, or magnetic resonance imaging (MRI) and may contain barium, iodine, manganese, iron, lanthanum, cerium, or gadolinium. Non-limiting examples of contrast agents include iodinated X-ray contrast agents such as iodixanol, iopromide, iohexol, iopamidol; and paramagnetic chelates such as gadolinium-DPTA (diethylenetriamine penta-acetic acid) or gadobutrol.

The fatty acids can be in triglyceride form. Non-limiting examples of polyunsaturated fatty acids include omega-3 fatty acids, such as a-linolenic acid (ALA), eicosapentaenoic acid (EPA), and docosahexaenoic acid (DHA). Other examples of additives or excipient materials that can be blended with the therapeutic agent include polyurethaneurea/heparin; polyurethane; or naturally occurring materials (e.g., collagen, laminin, heparin, fibrin, or cellulose).

Medical devices of the present invention may also include a vascular stent mounted on the balloon. The vascular stent may be any of those known in the art, including those with or without coatings that elute a therapeutic agent. The stent may also be biostable, bioerodable, or biodegradable. The stent may be a bare stent or may have a drug coating.

The balloons of the present invention may also be coated with a low-molecular weight carbohydrate, such as mannitol. The carbohydrate may be a separate coating or be blended with the therapeutic agent. The balloons of the present invention may also be coated with a radio contrast agent (ionic or non-ionic), such as iopromide, bismuth subcarbonate, bismuth oxychloride, bismuth trioxide, barium sulfate, tungsten, and mixtures thereof. The contrast agent may be a separate coating or be blended with the therapeutic agent. The balloons of the present invention may also be coated with a water-soluble polymer, such as polyvinylpyrrolidone (PVP). The polymer may be a separate coating or be blended with the therapeutic agent.

The therapeutic agent used in the present invention may be any pharmaceutically acceptable agent (such as a drug), a biomolecule, a small molecule, or cells. Exemplary drugs include anti-proliferative agents such as paclitaxel, sirolimus (rapamycin), tacrolimus, everolimus, biolimus, and zotarolimus. Exemplary biomolecules include peptides, polypeptides and proteins; antibodies; oligonucleotides; nucleic acids such as double or single stranded DNA (including naked and eDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Exemplary small molecules include hormones, nucleotides, amino acids, sugars, and lipids and compounds have a molecular weight of less than IOOkD. Exemplary cells include stem cells, progenitor cells, endothelial cells, adult cardiomyocytes, bone marrow cells, and smooth muscle cells. Other therapeutic agents that may be used in the present invention include those listed in U.S. Patent No. 7,572,625 (Davis et aI., "Medical devices coated with drug carrier macromolecules"). Any of the therapeutic agents may be combined to the extent such combination is biologically compatible.

## Claims

1. A medical device comprising:
a non-compliant balloon (14) that is folded into a compact configuration;
an expandable sheath (12) that encloses the balloon (14) in its compact configuration; and
a coating (16) disposed over the expandable sheath (12), the coating (16) comprising a therapeutic agent, and
wherein the coating is less compliant than the expandable sheath (12) such that the coating (16) breaks apart and delaminates as the sheath (12) expands when the balloon (14) is inflated.

2. The medical device of claim 1, wherein the expandable sheath (12) comprises a low adhesion material.

3. The medical device of claim 2, wherein the low adhesion material is a fluoropolymer.

4. The medical device of claim 1, further comprising an intermediate release layer disposed between the expandable sheath (12) and the therapeutic agent coating (16).

5. The medical device of claim 1, wherein the expandable sheath (12) experiences at least a 1.5- fold increase in diameter as the balloon (14) is fully inflated.

6. The medical device of claim 1, wherein the length of the sheath (12) is shorter than the length of the balloon (14).

7. The medical device of claim 6, wherein the length of the sheath (12) is 20 - 80% of the length of the balloon (14), and one or both ends of the balloon (14) are not covered by the sheath (12).

8. The medical device of claim 1, wherein the therapeutic agent (16) is in a crystalline form.

## Patentansprüche

1. Ein Medizingerät, umfassend:
einen nicht nachgebenden Ballon (14), der in eine kompakte Konfiguration gefaltet ist;
eine expandierbare Umhüllung (12), die den Ballon (14) in seiner kompakten Konfiguration umschließt; und
eine Beschichtung (16), welche über der expandierbaren Umhüllung (12) angeordnet ist, wobei die Beschichtung (16) ein therapeutisches Mittel umfasst, und
wobei die Beschichtung weniger nachgibt als die expandierbare Umhüllung (12), so dass die Beschichtung (16) auseinanderbricht und mit der Expandierung der Umhüllung (12) bei der Inflatierung des Ballons (14) delaminiert.

2. Das Medizingerät gemäß Anspruch 1, wobei die expandierbare Umhüllung (12) ein wenig haftendes Material umfasst.

3. Das Medizingerät gemäß Anspruch 2, wobei das wenig haftende Material ein Fluorpolymer ist.

4. Das Medizingerät gemäß Anspruch 1, weiter umfassend eine zwischenangeordnete Freisetzungsschicht, die zwischen der expandierbaren Umhüllung (12) und der Beschichtung mit dem therapeutischen Mittel (16) angeordnet ist.

5. Das Medizingerät gemäß Anspruch 1, wobei die expandierbare Umhüllung (12) mindestens eine 1,5-fache Steigerung im Durchmesser bei vollständiger Inflatierung des Ballons (14) erfährt.

6. Das Medizingerät gemäß Anspruch 1, wobei die Länge der Umhüllung (12) kürzer ist als die Länge des Ballons (14).

7. Das Medizingerät gemäß Anspruch 6, wobei die Länge der Umhüllung (12) 20-80% der Länge des Ballons (14) beträgt, und eine oder beide Enden des Ballons (14) nicht von der Umhüllung (12) bedeckt sind.

8. Das Medizingerät gemäß Anspruch 1, wobei das therapeutische Mittel (16) in einer kristallinen Form vorliegt.

## Revendications

1. Dispositif médical comprenant :
un ballonnet non flexible (14) qui est plié en une configuration compacte ;
une gaine dilatable (12) qui enveloppe le ballonnet (14) dans sa configuration compacte ; et
un revêtement (16) disposé sur la gaine dilatable (12), le revêtement (16) comprenant un agent thérapeutique, et
dans lequel le revêtement est moins flexible que la gaine dilatable (12) de telle sorte que le revêtement (16) se rompt et se sépare à mesure que la gaine (12) se dilate lorsque le ballonnet (14) est gonflé.

2. Dispositif médical selon la revendication 1, dans lequel la gaine dilatable (12) comprend un matériau à faible adhérence.

3. Dispositif médical selon la revendication 2, dans lequel le matériau à faible adhérence est un polymère fluoré.

4. Dispositif médical selon la revendication 1, comprenant en outre une couche antiadhésive intermédiaire disposée entre la gaine dilatable (12) et le revêtement (16) d'agent thérapeutique.

5. Dispositif médical selon la revendication 1, dans lequel la gaine dilatable (12) voit son diamètre multiplié par au moins 1,5 lorsque le ballonnet (14) est entièrement gonflé.

6. Dispositif médical selon la revendication 1, dans lequel la longueur de la gaine (12) est inférieure à la longueur du ballonnet (14).

7. Dispositif médical selon la revendication 6, dans lequel la longueur de la gaine (12) est égale à 20 à 80 % de la longueur du ballonnet (14), et l'une des extrémités du ballonnet (14) n'est pas couverte par la gaine (12) ou aucune des extrémités du ballonnet (14) n'est couverte par la gaine (12).

8. Dispositif médical selon la revendication 1, dans lequel l'agent thérapeutique (16) se présente sous forme cristalline.
